# EUROPEAN PATENT SPECIFICATION

(11) **EP 1 364 617 B1**
(45) Date of publication and mention of the grant of the patent: **10.08.2005**
(21) Application number: 03018620.9
(22) Date of filing: 22.10.1997
(51) Int. Cl.: A61B 17/02, A61B 17/16, A61F 2/44, A61F 2/46

(54) **Surgical drill and retractor**
Chirurgische Bohrer und Retraktor
Perceuse et écarteur chirurgicaux

(30) Priority: 22.10.1996 US 734911; 24.03.1997 US 822530
(43) Date of publication of application: 26.11.2003
(62) Divisional of application: 97912829.5
(73) Proprietor: Howmedica Osteonics Corp., Mahwah, NJ 07430 (US)
(72) Inventor: Winslow, Charles J., Walnut Creek, CA 94595 (US)
(74) Representative: Liebetanz, Michael, Dipl.-Phys.

(56) References cited:
- EP-A- 0 077 159
- EP-A- 0 307 241
- EP-A- 0 641 547
- EP-A- 0 716 840
- EP-A- 0 796 593
- WO-A-95/22946
- WO-A-95/32673
- WO-A-95/35180
- WO-A-96/27321
- WO-A-96/27339
- WO-A-96/27345
- US-A- 5 015 247
- US-A- 5 026 373
- US-A- 5 062 845
- US-A- 5 458 638
- US-A- 5 489 307
- US-A- 5 554 191

## Description

The present invention generally relates to a system for drilling a bore in adjacent verte-brae to facilitate the insertion of a fusion implant and, in particular, to instrumentation for insertion of spinal implants to facilitate fusion of adjacent vertebral bodies according to the preamble of claim 1.

### 2. Background of the Related Art

Such a system is known from WO 96/27 345 as explained in detail below.

The fusion of adjacent bone structures is commonly performed to provide for long-term replacement to compensate for degenerative or deteriorated disorders in bone. For example, an intervertebral disc, which is a ligamentous cushion disposed between adjacent vertebrae, may undergo deterioration as a result of injury, disease, tumor or other disorders. The disk shrinks or flattens leading to mechanical instability and painful disc translocations.

Conventional procedures for disc surgery include partial or total excision of the injured disc portion, e.g., discectomy, and replacement of the excised disc with biologically acceptable plugs or bone wedges. The plugs are driven between adjacent vertebrae to maintain normal intervertebral spacing and to achieve, over a period of time, bony fusion with the plug and opposed vertebrae. For example, U.S. Patent No. 4,887,020 to Vich discloses a threaded cylindrical bone plug which is screwed into a correspondingly dimensioned cylindrical bore drilled in the intervertebral space. Other bone grafting plugs are disclosed in U.S. Patent No. 4,950,296.

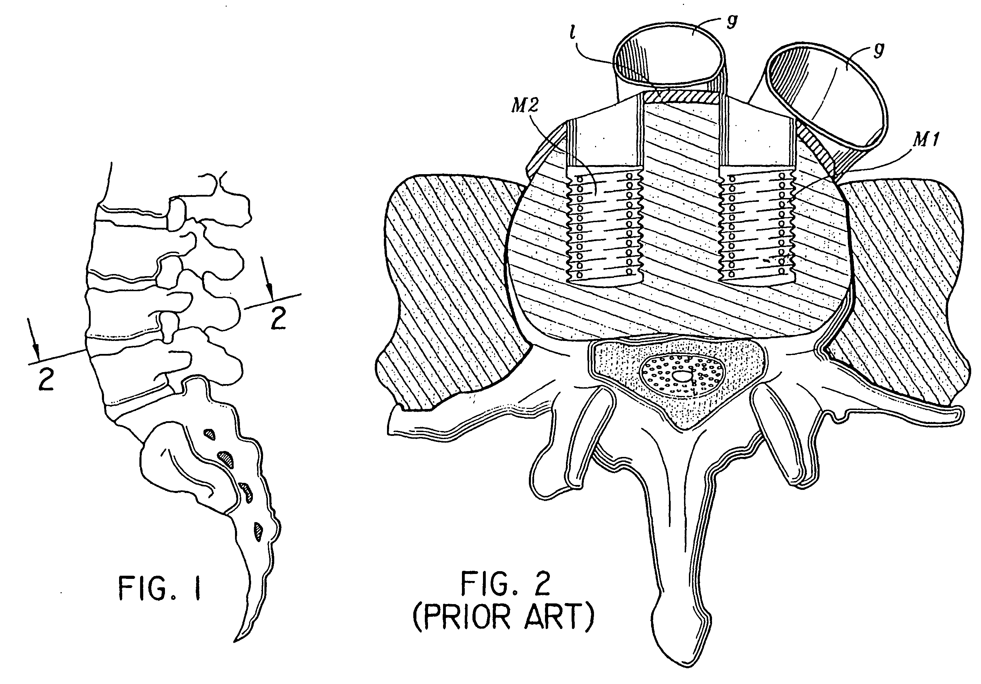

More recently, emphasis has been placed on fusing bone structures (i.e., adjoining vertebrae) with prosthetic cage implants. One fusion cage implant is disclosed in commonly assigned U.S. Patent No. 5,026,373 to Ray et al.. The Ray '373 fusion cage includes a cage having a thread formed as part of its external surface and apertures extending through its wall which communicate with an internal cavity of the cage body. The fusion cage is inserted within a tapped bore or channel formed in the intervertebral space thereby stabilizing the vertebrae and maintaining a pre-defined intervertebral space. Preferably, a pair of fusion cages are implanted within the intervertebral space. The adjacent vertebral bone structures communicate through the apertures and with bone growth inducing substances which are within the internal cavity to unite and eventually form a solid fusion of the adjacent vertebrae. FIGS. 1-2 illustrate the insertion of a pair of the Ray '373 fusion cages positioned within an intervertebral space.

Both anterior (transabdominal) and posterior surgical approaches are used for interbody fusions of the lumbar spine. Fusions in the cervical area of the spine are also performed using an anterior or a posterior approach. Typically, an implant such as a plug, dowel, prosthesis or cage is inserted into a preformed cavity inside the interbody, interdiscal space. Since it is desirable in these procedures to promote a "bone to bone" bridge, connective tissue and at least a portion of the distal tissue is removed. Preferably, relatively deep cuts are made in the adjacent bones in order to penetrate into the softer, more vascularized cancellous region to facilitate bone growth across the implant.

One of the more critical tasks performed in the insertion of a surgical fusion implant, particularly, in intervertebral spinal fusion, is the formation of the implant receiving cavity or bore within the adjacent vertebrae. More particularly, the drilled bore should be centered with respect to and preferably parallel to the vertebral end plates to ensure removal of equal portions of bone from the adjacent vertebrae throughout the length of the cut and subsequent appropriate seating of the implant relative to the vertebral bodies.

Surgical instruments for spinal fusion implant insertion are known. For example, U.S. Patent No. 5,484,437 to Michelson discloses a method and apparatus incorporating an outer and an inner sleeve arrangement. The outer sleeve has teeth at one end which are driven directly into the posterior surface of the adjacent vertebrae. The inner sleeve is positioned within the outer sleeve and serves to guide instruments such as a drill used to form the implant receiving bore. U.S. Patent Nos.: 5,487,307 to Kuslich et al.; 5,015,247 to Michelson; and 4,878,915 to Brantigan disclose similar arrangements. Other arrangements include the use of guide rods which are placed in pilot holes formed in the vertebral bodies. The guide rods guide a bore forming hollow drill into the intervertebral space.

Although some of the current instrumentation and methods associated therewith for enhancing the placement of spinal fusion implants have been generally effective for their intended purposes, there exists certain limitations with the design of this instrumentation which detract from their usefulness. For example, the arrangement disclosed in the Michelson '437 patent and similar arrangements do not provide for automatic alignment of the outer sleeve to ensure that the bore formed by a drill introduced into the outer sleeve is in optimal alignment for a tapping procedure (if required) and reception of the spinal implant. Rather, such orientation is dependent directly upon the skill of the surgeon. Moreover, the outer sleeve, which is only mounted only at its extreme distal end to the posterior surface of the adjacent vertebrae, is subject to disorientation or dislodgment during insertion and/or removal of the drill and/or tapping instrument. Similarly, the use of guide rods increases the number of steps required to implant the fusion cage and is also subject to possible misalignment.

U.S. Patent Application Serial No. 08/615,379, filed March 14, 1996, discloses a method and associated instrumentation to facilitate the introduction of a fusion implant. The instrumentation disclosed in the '379 application ensures optimal alignment of the drilled bore for reception of the fusion implant and, if appropriate, for bore tapping procedures. The instrumentation includes a surgical retractor and a drill. The retractor is configured for distracting adjacent vertebral bodies to facilitate the insertion and application of an implant, for providing a cannula for insertion of auxiliary instruments, e.g., the drill, and for ensuring proper alignment of the instrumentation and accurate insertion of the implant. The instrumentation and method disclosed in the '379 application is well suited for implanting an implant having a general circular cross-sectional portion such as the aforedescribed Ray '373 fusion cage.

### SUMMARY

WO 96/27 345 discloses a system for drilling a bore in adjacent vertebrae to facilitate the insertion of a fusion implant, comprising a surgical retractor including a sleeve member having proximal and distal end portions and defining a longitudinal opening, the distal end portion configured for insertion at least partially into an intervertebral space between adjacent opposed vertebrae to distract the adjacent vertebrae, and a drill instrument positionable within the longitudinal opening of the surgical retractor and including a drill member having a distal cutting head. It is therefore possible to guide a drill instrument within an sleeve member of an retractor.

However, this prior art does not enable the surgeon to fine tune his drilling action when he advances into the intervertebral space.

The present disclosure is directed to a system for drilling a bore in adjacent vertebrae to facilitate the insertion of a fusion implant comprising a surgical retractor including a sleeve member having proximal and distal end portions and defining a longitudinal opening, with the distal end portion configured for insertion at least partially into an intervertebral space between adjacent opposed vertebrae to distract the adjacent vertebrae and the sleeve member including an internal threaded portion. A drill instrument is positionable within the longitudinal opening of the surgical retractor, and includes a drill member having a distal cutting head and an external threaded portion engageable with the internal threaded portion of the retractor whereby rotation of the drill instrument causes distal translation of the drill instrument relative to the surgical retractor.

An apparatus for facilitating fusion of adjacent vertebrae is also disclosed. The apparatus includes an implant member configured and dimensioned for insertion within an intervertebral space defined between adjacent vertebrae. The implant member includes at least a longitudinal section having a transverse cross-sectional dimension defining a generally elliptical configuration. The implant member includes an internal cavity for accommodating bone growth inducing substances and a plurality of apertures extending through an external wall portion thereof in communication with the internal cavity. An external threaded portion is formed on the implant member for facilitating insertion within the intervertebral space.

A system and associated method to facilitate insertion of the fusion implants is also disclosed. In a preferred embodiment, a system for drilling a bore in adjacent vertebrae to facilitate the insertion of a fusion implant includes a surgical retractor including a sleeve member with proximal and distal end portions and defining a longitudinal opening and a drill instrument positionable within the longitudinal opening of the surgical retractor. The distal end portion of the retractor is configured for insertion at least partially into an intervertebral space between adjacent opposed vertebrae to distract the adjacent vertebrae to a desired predetermined distracted position.

Preferably, the drill instrument includes an elongate member having a longitudinal passageway and defining at least one distal cutting surface and a drill member disposed within the elongate member and having a distal drill head. The drill member is rotatably movable within the elongate member and is also longitudinally fixed to the elongate member such that advancement of the drill member within the retractor causes corresponding advancement of the elongate member such that the distal cutting surface of the elongate member and the distal drill head of the drill member cooperate to cut a bore, e.g., an elliptical-shaped bore, in the adjacent vertebrae. Preferably, the elongate member of the drill instrument includes first and second diametrically opposed distal cutting surfaces. The cutting surfaces may be arcuately-shaped.

Preferably, the distal end portion of the retractor includes two spaced apart retractor arms having first and second support surfaces which respectively engage and distract upper and lower vertebrae. At least one anchoring member may be associated with the surgical retractor to facilitate mounting of the retractor to the vertebrae.

The system may further include alignment means for aligning and maintaining the elongate member of the drill instrument at a predetermined angular orientation within the sleeve member of the surgical retractor. The preferred alignment means is adapted to angularly orient the first and second distal cutting surfaces in general alignment within respective retractor arms of the surgical retractor. The alignment means may include at least one groove defined in the sleeve member of the surgical retractor, the one groove dimensioned to accommodate a corresponding spline of the elongate member.

A method for performing a surgical procedure with the system is also disclosed. The method includes the steps of providing a surgical retractor including an elongate member defining a longitudinal opening and having two spaced apart retractor arms with first and second supporting surfaces at its distal end, inserting the retractor arms of the retractor within the intervertebral space whereby the first and second supporting surfaces of each retractor arm respectively engage and distract the adjacent opposed vertebras, mounting the surgical retractor to the adjacent vertebrae by securing an anchor member associated with the surgical retractor to the adjacent vertebrae and performing the surgical procedure adjacent the distracted vertebrae by, e.g., introducing surgical instrumentation within the opening of the surgical retractor.

A method for fusing adjacent vertebral bodies with the system is also disclosed. The method includes the steps of accessing the intervertebral disc space, providing a retractor including a retractor sleeve having opposed retractor arms extending in a general longitudinal direction, positioning the retractor arms within the intervertebral disc space whereby first and second supporting surfaces of each arm contact opposed vertebra bodies, introducing a drill instrument into the retractor sleeve and advancing the drill instrument within the sleeve to the disc space wherein the drill instrument includes an elongate member having a longitudinal passageway and defining at least one distal cutting surface and a drill member rotatably mounted within the elongate member and having a distal cutting head, actuating the drill instrument such that the distal cutting head of the drill member and the distal cutting surface of the elongate member are advanced into the adjacent vertebrae to cooperate and cut a bore in the adjacent vertebra, removing the drill instrument from the sleeve, and introducing a fusion implant into the bore. Preferably an elliptical bore is formed and a fusion implant having an elliptical cross-sectional dimension is inserted into the bore.

### BRIEF DESCRIPTION OF THE DRAWINGS

Preferred embodiment(s) of the present disclosure are described herein with reference to the drawings wherein:
FIG. 1 is a view illustrating a portion of the vertebral column of a patient;
FIG. 2 is a view taken along line 2-2 of FIG. 1 illustrating a pair of prior art fusion implants positioned within the intervertebral space for fusion of adjacent vertebrae;
FIGS. 3-4 are front and rear perspective views of the fusion implant in accordance with the principles of the present disclosure;
FIG. 5 is a perspective view of the fusion implant of FIGS. 3-4 illustrating the implant body and detachable end cap;
FIG. 6 is a side plan view of the implant body;
FIG. 7A is an axial view taken along line 7A-7A of FIG. 6 illustrating the entry end portion of the implant body;
FIG. 7B is an axial view taken along lines 7B-7B of FIG. 6 illustrating the trailing end portion of the fusion implant;
FIG. 8 is a side cross-sectional view of the implant body and mounted end cap taken along line 8-8 of FIG. 7B;
FIG. 9 is a top cross-sectional view of the implant body and mounted end cap taken along line 9-9 of FIG. 7B;
FIG. 10A is a cross-sectional view taken along line 10A-10A of FIG. 9 illustrating a section through the major diameter of the thread;
FIG. 10B is a cross-sectional view taken along line 10B-10B of FIG. 9 illustrating a section through the minor diameter of the thread;
FIG. 11 is a cross-sectional view taken along line 11-11 of FIG. 9 illustrating the circular configuration of the entry end portion of the implant body;
FIG. 12 is a perspective view of an instrumentation kit utilized for inserting the fusion implant within the intervertebral space, including a surgical retractor, a surgical drill, an implant insertion instrument and a T-shaped handle;
FIG. 13 is a view illustrating the lateral insertion of the surgical retractor within the intervertebral space;
FIG. 14 is a view taken along line 14-14 of FIG. 13 further illustrating positioning of the retractor within the intervertebral space and engagement of the retractor with the vertebral end faces of the adjacent vertebrae;
FIG. 15 is a view similar to the view of FIG. 14 illustrating insertion of a drilling instrument into the retractor to drill a bore within the adjacent vertebrae;
FIG. 16 is a side plan view illustrating the insertion instrument with the fusion implant mounted to the insertion instrument;
FIG. 16A is a cross-sectional view of the distal end of the insertion instrument and the fusion implant illustrating mounting of the end cap to the implant body;
FIG. 17 is a view similar to the view of FIG. 15 illustrating insertion of the insertion instrument and mounted implant through the retractor;
FIGS. 18-20 are enlarged views illustrating positioning of the fusion implant within the preformed bore;
FIG. 21 is a view illustrating the fusion implant mounted within the intervertebral space;
FIG. 22 is a sectional view further illustrating the fusion implant mounted within the intervertebral space;
FIG. 23 is a view illustrating a different sized fusion implant mounted within the vertebral space;
FIG. 24 is a perspective view of an alternate system for inserting the implant of FIGS. 3-11 including a surgical retractor utilized in distracting adjacent bony structures and a surgical drilling instrument utilized in drilling a bore within the adjacent bony structure in accordance with the principles of the present disclosure;
FIG. 24A is a perspective view of an insertion instrument and detached T-handle utilized in inserting an implant within the adjacent bony structures;
FIG. 25 is a perspective view with parts separated of the surgical retractor of FIG. 24;
FIG. 26 is a side view in cross-section of the surgical retractor of FIG. 25;
FIG. 26A is an isolated view of the anchoring member being retained within the retractor;
FIG. 27 is an axial view of the surgical retractor;
FIG. 28 is a side plan view of the drilling instrument;
FIG. 29 is an isolated view in cross-section illustrating the mounting of the drill shaft and drill bit and the mounting of the extension sleeve and the drill shaft;
FIG. 30 is an axial view of the drilling instrument;
FIG. 31 is a view of portion of the vertebral column;
FIG. 32 is a sectional view of the vertebral column taking along the lines 31-31 of FIG. 31 illustrating insertion of the surgical retractor within the intervertebral space;
FIG. 33 is a cross-sectional view further illustrating the surgical retractor inserted within the intervertebral space;
FIG. 34 is a view similar to the view of FIG. 33 illustrating mounting of the anchoring screws into the vertebral column;
FIG. 35 is a view similar to the view of FIG. 34 illustrating insertion of the drilling instrument into the surgical retractor;
FIG. 36 is a view similar to the view of FIG. 35 illustrating advancement of the drilling instrument to drill a bore within adjacent vertebrae;
FIG. 36A is a cross-sectional view taken along the lines 36A-36A of FIG. 36;
FIG. 37 is a view similar to the view of FIG. 36 illustrating insertion of the insertion instrument and mounted fusion implant into the surgical retractor to insert the implant;
FIG. 38 is a sectional view illustrating the fusion implant mounted within the intervertebral space; and
FIG. 39 is a view further illustrating the fusion implant mounted within the intervertebral space.

### DETAILED DESCRIPTION OF PREFERRED EMBODIMENTS

The preferred embodiment of the apparatus and method disclosed herein are discussed in terms of orthopedic spinal fusion procedures and instrumentation. It is envisioned, however, that the disclosure is applicable to a wide variety of procedures including, but, not limited to ligament repair, joint repair or replacement, non-union fractures, facial reconstruction and spinal stabilization. In addition, it is believed that the present method and instrumentation finds application in both open and minimally invasive procedures including endoscopic and arthroscopic procedures wherein access to the surgical site is achieved through a cannula or small incision.

The following discussion includes a description of the fusion implant utilized in performing a spinal fusion followed by a description of the preferred method for spinal fusion in accordance with the present disclosure.

In the discussion which follows, the term "proximal", as is traditional, will refer to the portion of the structure which is closer to the operator, while the term "distal" will refer to the portion which is further from the operator.

### Fusion Implant

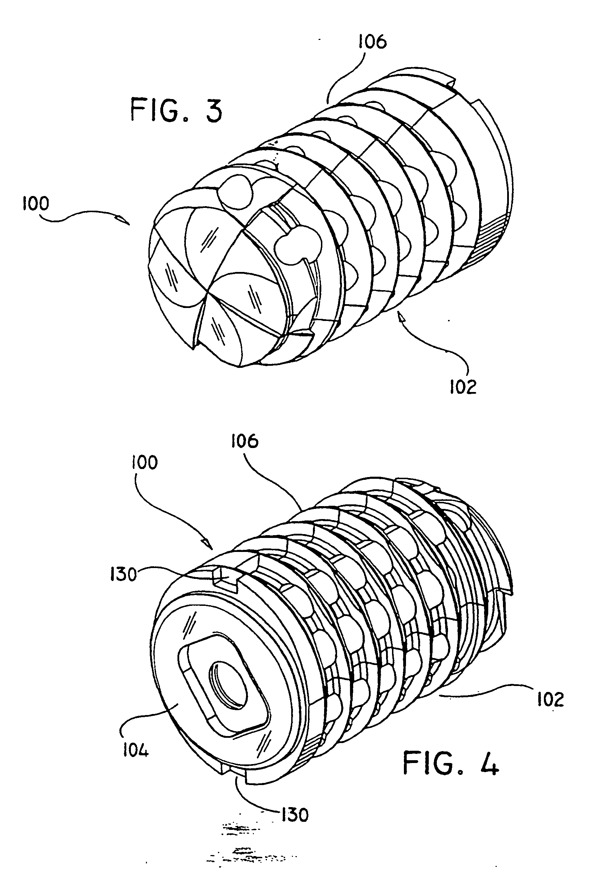

Referring now to the drawings in which like reference numerals identify similar or identical elements throughout the several views, FIGS. 3-5 illustrate in perspective the fusion implant of the present disclosure. Fusion implant 100 is contemplated to be a self-tapping implant, i.e., the implant is intended to be inserted within a preformed bore in adjacent bone structures, e.g., adjacent vertebrae, without necessitating tapping of an internal thread within the bone structures prior to insertion and is preferably configured for lumbar vertebrae. Fusion implant 100 includes elongated implant body 102 and end cap 104 which is mountable to the implant body 102. Implant body 102 is preferably fabricated from a suitable biocompatible rigid material such as titanium and/or alloys of titanium, stainless steel, ceramic materials or rigid polymeric materials. Implant body 102 is preferably sufficient in strength to at least partially replace the supporting function of an intervertebral disc, i.e., to maintain adjacent vertebrae in desired spaced relation, during healing and fusion, and is strategically dimensioned to span the intervertebral space such that only one implant (as opposed to two as is conventional) is required for insertion. The implant 100 is preferably provided in various lengths such as about 24 mm, 26 mm and 28 mm for example.

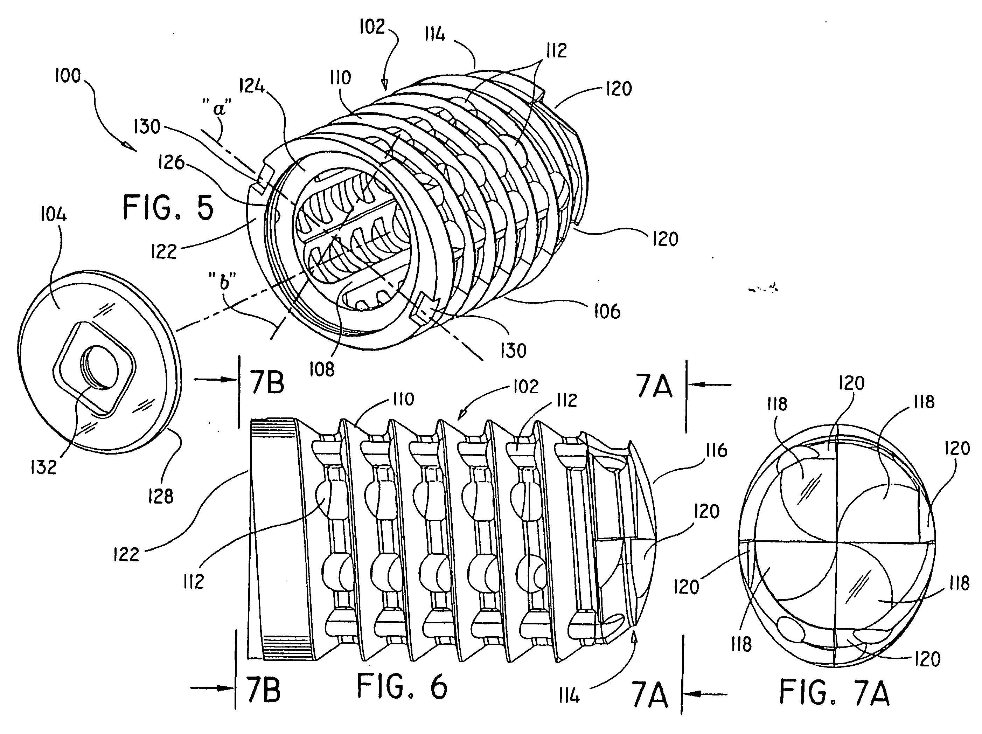

As best depicted in FIGS. 5-7B, implant body 102 is generally elliptical in configuration defining a major axis "a" greater than a minor axis "b" (FIG. 5). This configuration provides a greater surface area of the implant so as to facilitate contacting engagement and support of the implant with the adjacent vertebrae. In particular, as discussed in greater detail hereinbelow, in the inserted position of the fusion implant 100, the major axis "a" is in general parallel relation with the vertebral end faces of the adjacent vertebrae, thus, positioning the major arc or outer surface of implant body 102 in contact with the vertebral end faces. The oval or elliptical configuration and dimensions enable one implant to be utilized instead of two implants of the prior art. The elliptical configuration of implant body 102 also minimizes any tendency of the inserted implant 100 from backing out of the preformed bore. Implant body 102 includes an outer wall 106 which enclose an inner cavity 108 defined within the interior of the implant body 102. Inner cavity 108 accommodates bone growth inducing substances which facilitate the fusion process.

In a preferred embodiment, the diameter of the implant 102 along its major axis preferably ranges from about 16 mm to about 20 mm, and preferably is about 19 mm. The diameter along the minor axis preferably ranges from about 14 mm to about 17 mm, and preferably is about 16 mm. Other dimensions are also contemplated.

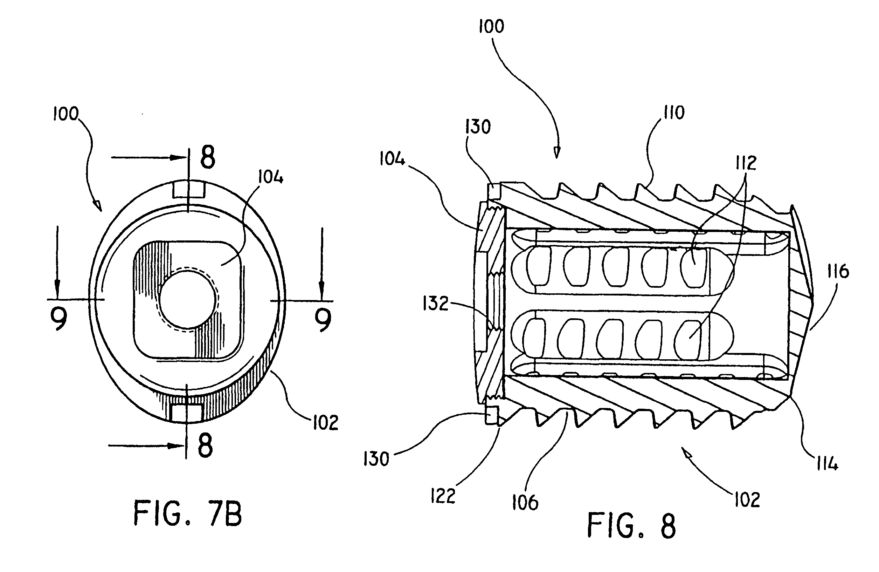

With reference to FIGS. 8-10B, in conjunction with FIG. 5, outer wall 106 has an external threaded configuration formed as part of its exterior surface. External threaded configuration including a continuous helical thread 110 which assists in advancing implant body 102 into a preformed channel provided in the adjacent vertebrae. Thread 110 as shown preferably has an angled face on the posterior side and a sharp end toward the anterior side to prevent expulsion to the anterior side. Thread 110 is preferably a self-tapping cutting thread, i.e., the threads are capable of deburring bone material during advancement into the performed channel. Alternatively, a thread can be tapped in the bone prior to insertion of the implant.

A plurality of apertures 112 extend through outer wall 106 of implant body 102. Apertures 112 are preferably formed by broaching grooves in the internal surface of the internal cavity 108. The effect of such broaching is to remove material from the valleys between the threads 110, thus defining the apertures 112. The advantages of such an arrangement are disclosed in U.S. Patent No. 4,961,740, and include immediate bone to bone contact between the vertebral bodies or bone structures and the bone inducing substances packed within the internal cavity 108 of the implant body 102. Apertures 112 are preferably substantially the same in dimension although it is envisioned that the dimensions of the apertures may vary to provide for more or less bone to bone contact as desired.

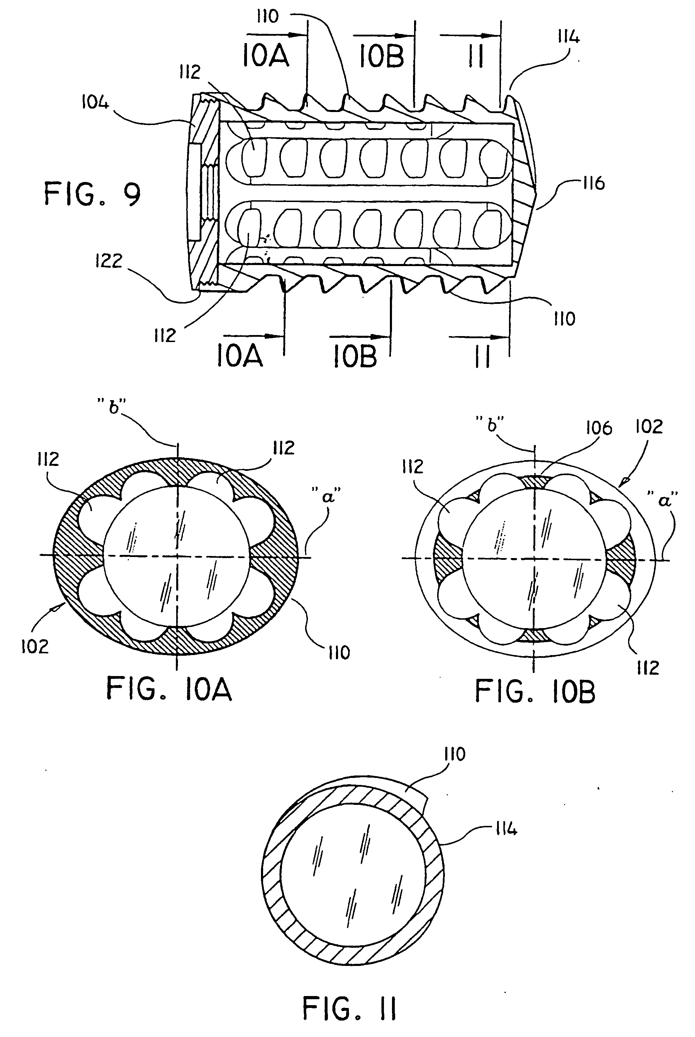

As best depicted in FIGS. 10A-10B, apertures 112 are clustered about a transverse axis or minor axis "b", both at the upper and lower end of the axis. Consequently, apertures 112 come into contact with the upper and lower vertebral bone structures to encourage bone growth through implant body 102 from the vertebral bone structures. The lateral sections of implant body 102 formed along the major axis "a" do not have apertures in order to prevent growth of disk material which might interfere with the bone fusing process.

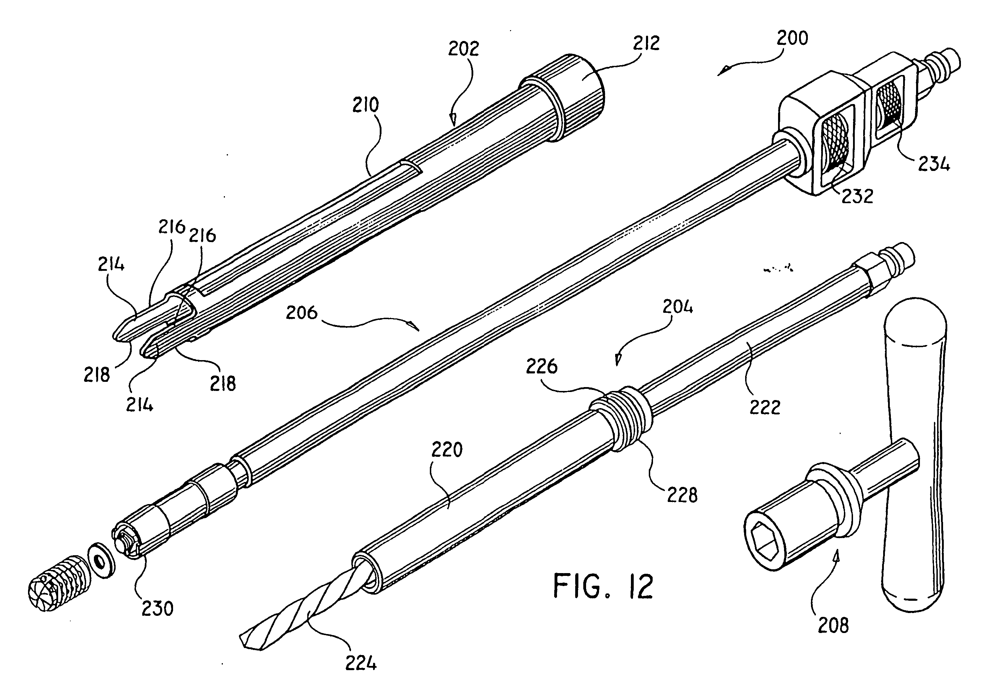

With reference now to FIGS. 6-7A and 11, the entry or leading end potion (distal) 114 of implant body 102 is preferably rounded, i.e., generally circular in cross-section as best depicted in FIG. 11 and defines a closed rounded entry end surface 116. This facilitates insertion. End surface 116 includes a plurality of flutes or relief grooves 118 formed in its surface. (four are shown). Flutes 118 assist in insertion of fusion implant 100 within the intervertebral space by capturing bone material deburred during the self-tapping process. In a preferred embodiment, flutes 118 meet at a central point of the longitudinal axis on the entry end of surface 116 and extend proximally to at least the first turn of the thread on implant body 102. The flute portions formed on thread 110 are defined by the sections 120 which are removed from the thread. (See also FIG. 5.) This arrangement permits adequate relief for purposes of self tapping of implant 100 in the intervertebral space. It is also envisioned that the flutes may run deeper and extend from the leading end 114 completely to the end cap 104, or, to a position intermediate the end cap 104 and the leading end 114.

With reference now to FIG. 5 and FIG. 7B, the trailing end portion 122 of implant body 102 has a generally annular recess 124 which receives end cap 104. An internal thread 126 is disposed adjacent annular recess 124 and cooperates with external thread 128 on the periphery of end cap 104 to mount the end cap to implant body 102. Trailing end portion 122 also includes a pair of diametrically opposed notches 130. Notches 130 are dimensioned to be engaged by corresponding structure of an insertion apparatus utilized in inserting the implant within the vertebral column. End cap 104 includes a central threaded aperture 132 which threadably engages corresponding structure of the insertion apparatus to assist in the mounting of the cap 104 on implant body 102.

### Instrumentation Kit

Referring now to FIG. 12, there is illustrated one preferred instrumentation kit for inserting spinal implant 100 within the intervertebral space. The instrumentation kit 200 includes surgical retractor 202, drill instrument 204 and insertion instrument 206. A T-shaped handle 208 is also provided in kit 200, and is utilized to actuate drill instrument 204 and insertion instrument 206.

Surgical retractor 202 is disclosed in commonly assigned U.S. patent Application Serial No. 08/615,379, filed March 14, 1996. Retractor 202 is configured for distracting adjacent vertebral bodies to facilitate the insertion and application of an implant, for providing a cannula for insertion of the instruments, and for ensuring proper alignment of the instrumentation and accurate insertion of the implant. Retractor 202 includes sleeve 210 with an enlarged head 212 at the proximal end of the sleeve 210. Sleeve 210 includes first and second diametrically opposed retractor arms 214 having first and second parallel vertebrae supporting surfaces 216, 218.

Drill instrument 204 is also disclosed in the '379 application. Drill instrument 204 includes drill shaft 220, extension shaft 222 and drill bit 224 mounted at the distal end of the drill shaft. T-handle 208 is mountable to a proximal mounting section of the drill instrument 204. Extension shaft 222 has first and second collars 226, 228 which cooperate to control the depth of penetration of drill shaft 220 into the adjacent vertebrae.

Insertion instrument 206 is disclosed in commonly assigned U.S. patent Application Serial No. 08/616,120, filed March 14, 1996. Insertion instrument 206 includes implant engaging structure 230 at its distal end which is correspondingly configured to mount and release implant 100 as will be discussed herein below. A pair of control wheels 232, 234 serve to control actuation of insertion instrument 206 thereby controlling mounting and releasing of the implant within the intervertebral space.

### Insertion of Fusion Implant With Instrumentation Kit

The insertion of the fusion implant 100 with the instrumentation kit 200 into an intervertebral space defined between adjacent lumbar vertebrae will now be described. The subsequent description will be particularly discussed in conjunction with an open antero-lateral approach for spinal fusion implant insertion. However, it is to be appreciated that other approaches, e.g., posterior, direct anterior, etc... could be utilized. Laparoscopic approaches are also envisioned.

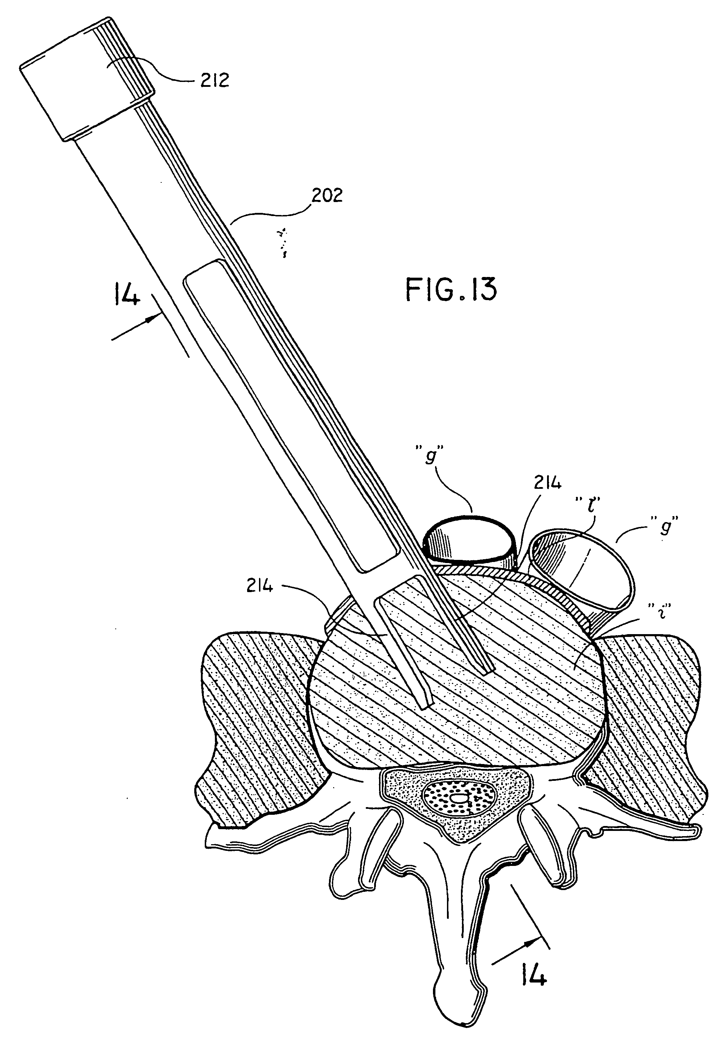

With respect now to FIG. 13, the intervertebral space "i" is accessed utilizing appropriate retractors to expose the anterior vertebral surface. Thereafter, retractor 202 is inserted within the intervertebral space "i" from an antero-lateral or oblique approach with relation to the vertebral columns 216, 218 as depicted in FIG. 13. Such approach provides advantages with regard to avoiding interference by the great vessels "g" (FIG. 13) and limiting penetration of the anterior longitudinal ligament "l". The retractor may be inserted by placing an impactor cap at the proximal end and impacting the retractor into the intervertebral space.

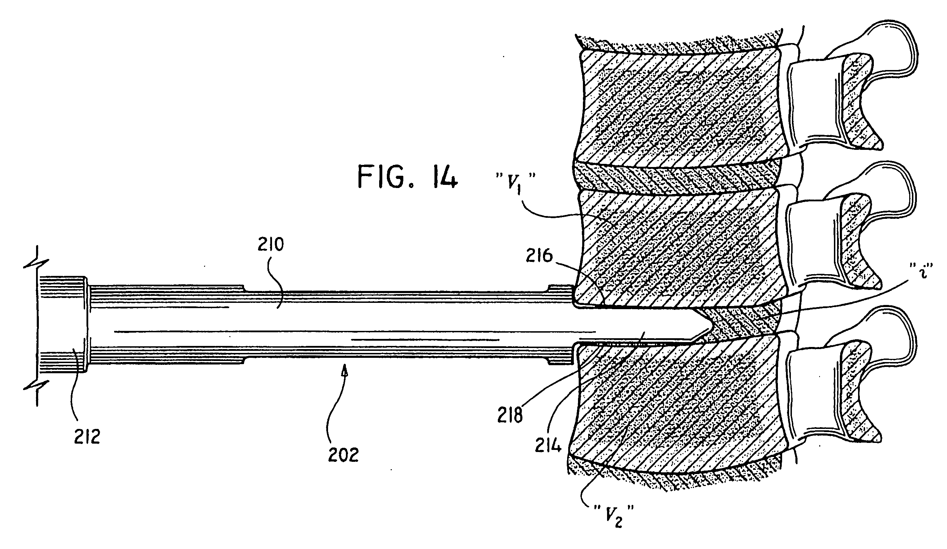

FIG. 14 depicts retractor 202 positioned within the intervertebral space "i" with the retractor arms 214 arranged such that the first and second supporting surfaces 216,218 of each retractor arm 214 respectively engage the opposed vertebral bodies "V₁, V₂". Upon insertion of retractor arms 214, the vertebral bodies "V₁, V₂" are distracted whereby the retractor arms become firmly lodged within the intervertebral space "i".

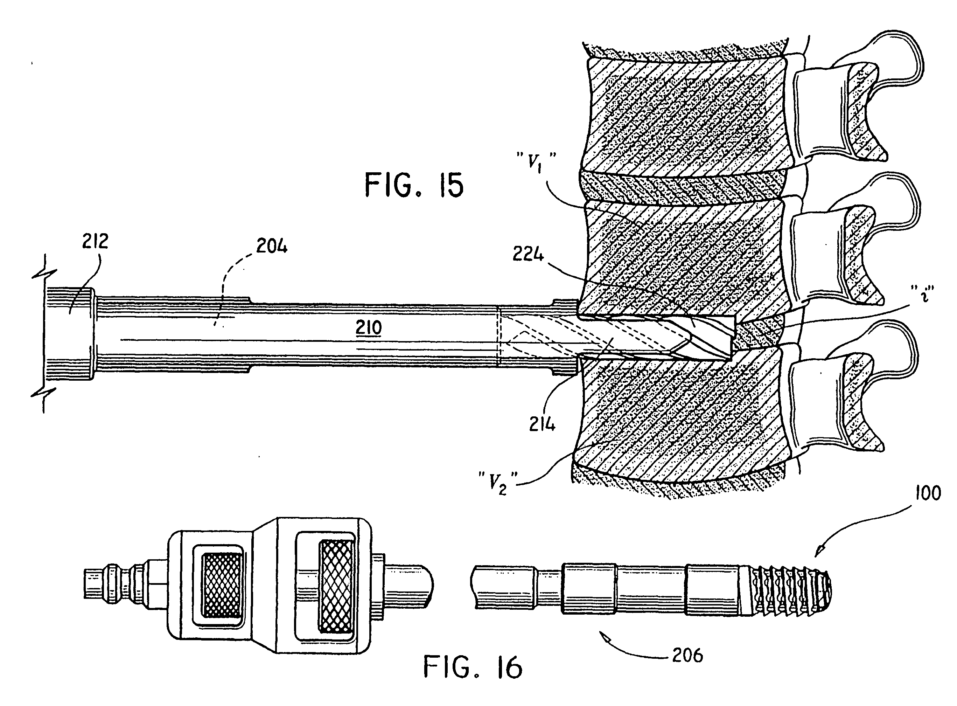

Referring now to FIG. 15, the drilling instrument 204 is now utilized to prepare the disc space and vertebral end plates for insertion of the fusion implant. The cutting depth of drilling instrument 204 is adjusted as desired (i.e., to correspond to the length of the fusion implant) by adjusting collars 226, 228. With the T-handle 208 mounted to drilling instrument 204, the instrument is introduced into retractor 202 and advanced to contact the anterior surface of the vertebral bodies "V₁ V₂". Drilling instrument 204 is advanced into the intervertebral space "i" by rotating T-handle 208 to shear the soft tissue and cut the bone of the adjacent vertebrae "V₁ V₂" thereby forming a bore which extends into the adjacent vertebrae "V₁, V₂".

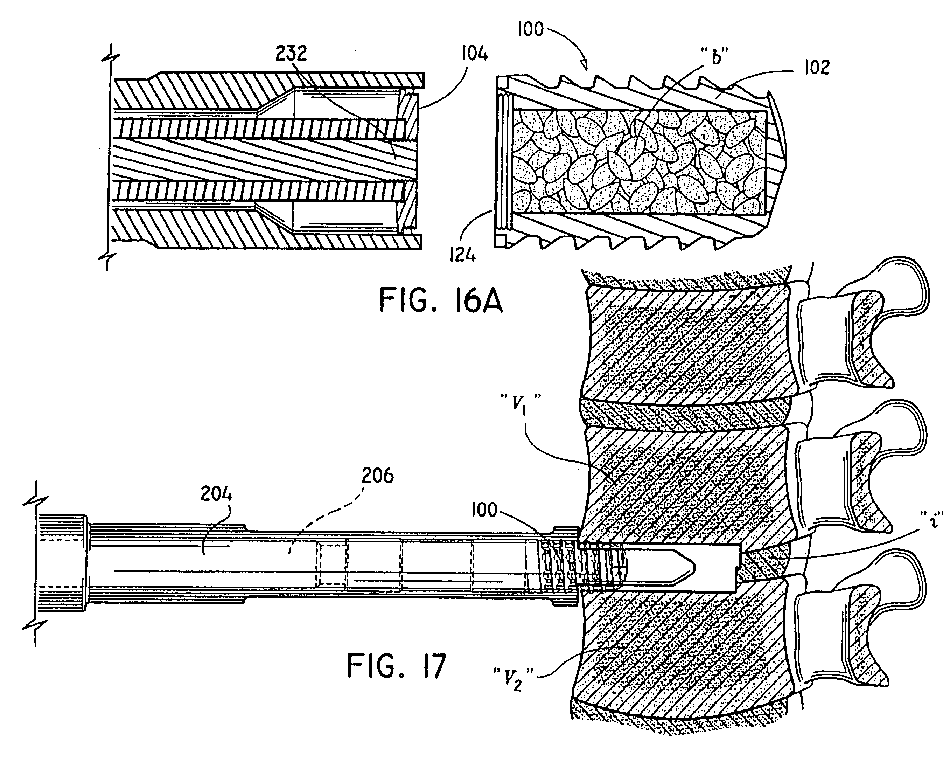

Subsequent to the drilling process, fusion implant 100 is packed with bone growth inducing substances "b" as in conventional in the art and end cap is threaded into recess 124 of implant body 102 either by hand, with a socket wrench-type instrument or with insertion instrument 206 as depicted in FIG. 16A. In particular, as shown in FIG. 16A, end cap 104 may be threaded onto mounting screw 232 of insertion instrument 206 and then threaded into recess 124 of implant body 102 via rotation of wheel 232. The fusion implant 100 is then mounted on insertion instrument 206 by positioning distal tabs 234 of insertion instrument 206 within correspondingly dimensional recesses 128 of end cap 104 (FIG. 5). FIG. 16 illustrates fusion implant 100 mounted to insertion instrument 206. Further details of the mounting of implant 100 to insertion instrument 206 may be ascertained by reference to the '120 application.

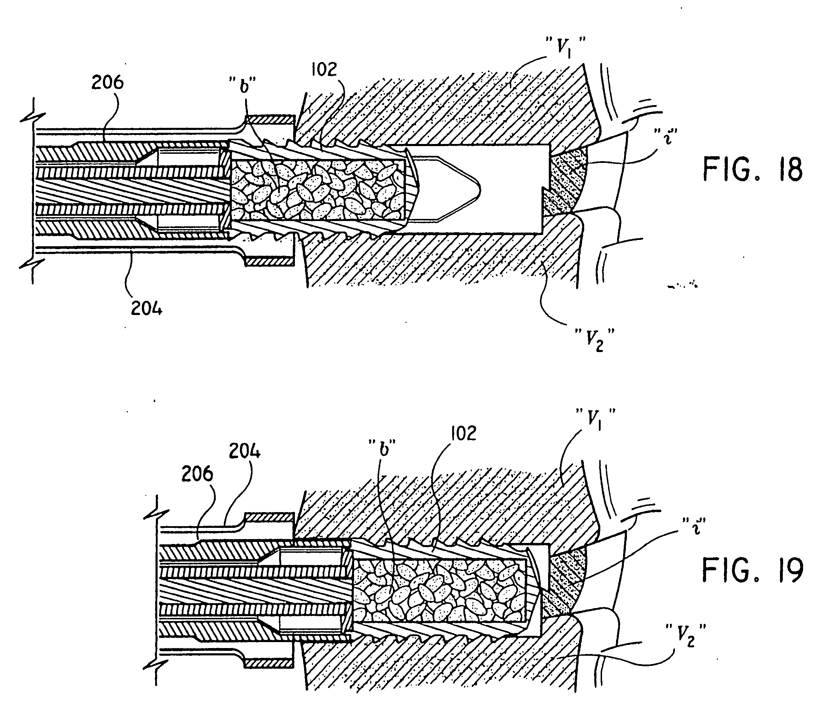

Referring now to FIG. 17, insertion instrument 206 and mounted implant 100 is introduced within retractor 204 and advanced to a position adjacent the vertebral bodies "V₁, V₂". Thereafter, insertion instrument 206 is rotated via T-shaped handle 202 which is mounted to the instrument 206 to thereby cause corresponding rotation of fusion implant 100. As fusion implant 100 rotates, the thread 110 of the implant body 102 bites into the vertebral bodies "V₁, V₂". Continued rotation of insertion tool 206 causes implant to move through the position shown in FIG. 18 to the position shown in FIG. 19 to be self-tapped within the preformed bore. Implant 100 is released from its mounting to insertion tool 206 and the instrument 206 and retractor 204 are removed from the disc area.

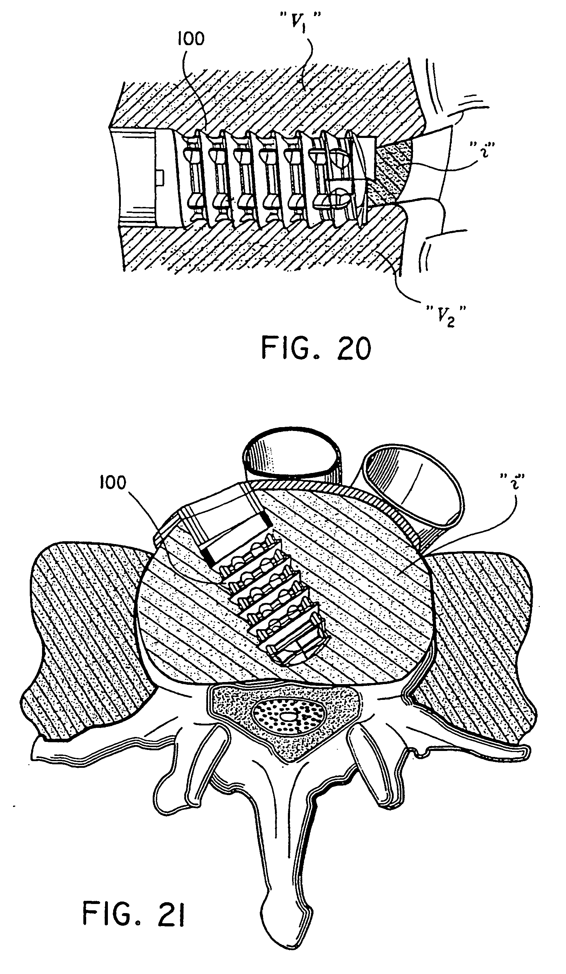

FIGS. 20-22 depict fusion implant 100 inserted within the intervertebral space "i". As shown, fusion implant 100 forms a strut across the intervertebral space "i" to maintain the adjacent vertebrae "V₁, V₂" in appropriate spaced relation during the fusion process. The implant is thus preferably inserted at an angle of between about 15 degrees and about 45 degrees, and more preferably at about 30 degrees to the longitudinal axis of the spine and to the right of the great vessels as view anteriorly. As also shown, in the inserted position of implant 100, the major axis "a" is in general parallel relation to the vertebral end plates thus presenting the greater arc or surface area of implant body 102 to contact and support the adjacent vertebrae. Over a period of time, the adjacent vertebral tissue communicates through apertures 112 with the bone growth inducing substances "b" within the interior cavity 108 of implant to form a solid fusion. Thus only one implant is required as opposed to two implants of the prior art as shown in Fig. 2. Implantation of the implants M1, M2 of Fig. 2 require greater manipulation due to the presence of the great vessels "g" and require increased penetration of the anterior longitudinal ligament "I".

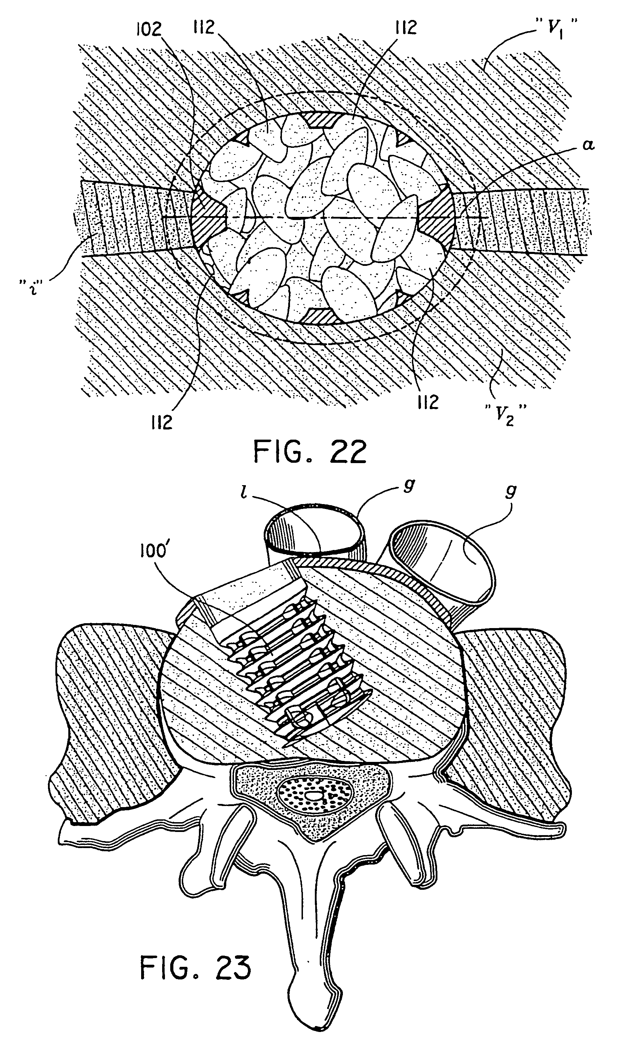

FIG. 23 by way of example illustrates a different sized fusion implant 100' positioned within the intervertebral space. This cage fills a larger portion of the disc space.

### Alternate Instrumentation Kit

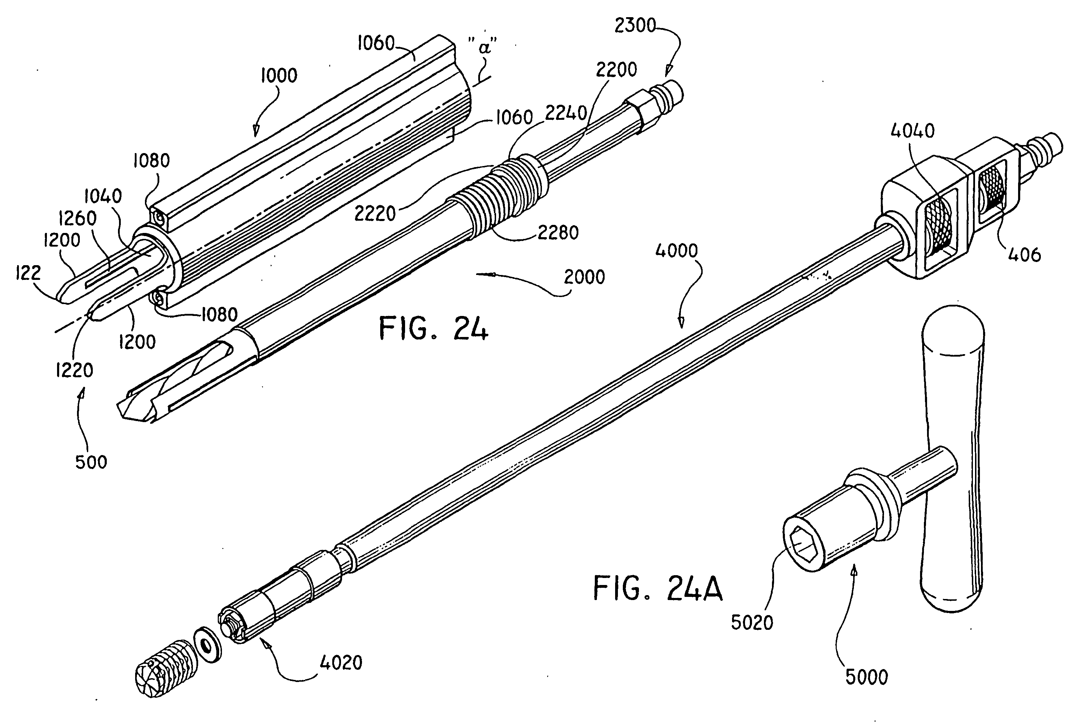

FIG. 24 illustrates, in perspective, an alternate surgical system or kit particularly contemplated for facilitating the insertion of the fusion implant of FIGS. 3-11 within the intervertebral space defined between adjacent vertebrae. System 500 generally includes two surgical instruments, namely retractor 1000 and drilling instrument 2000 which is positionable within the retractor 1000.

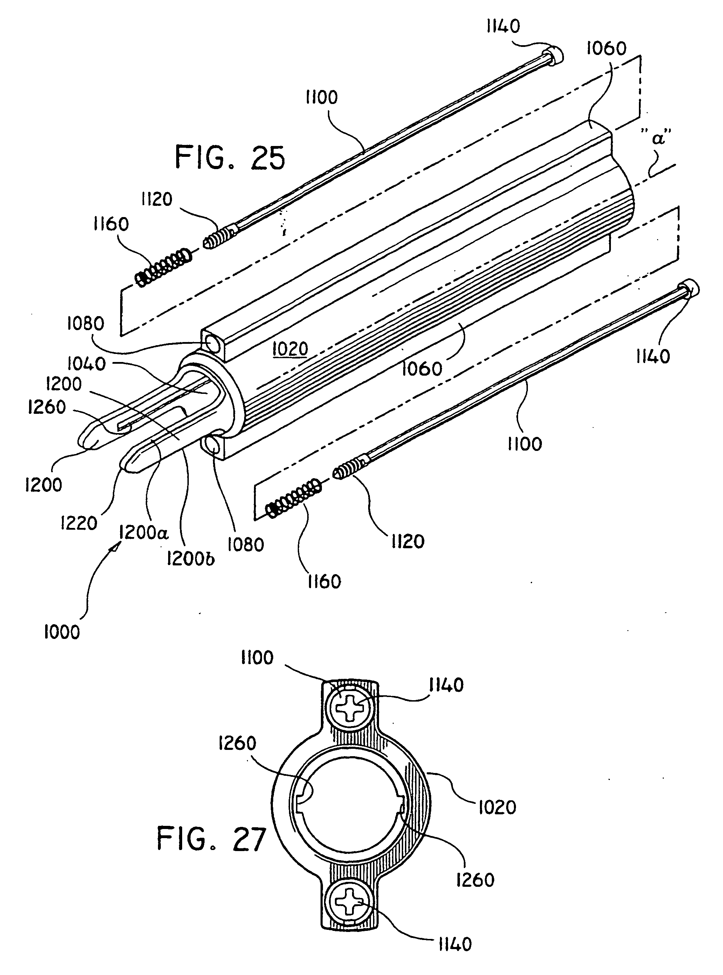

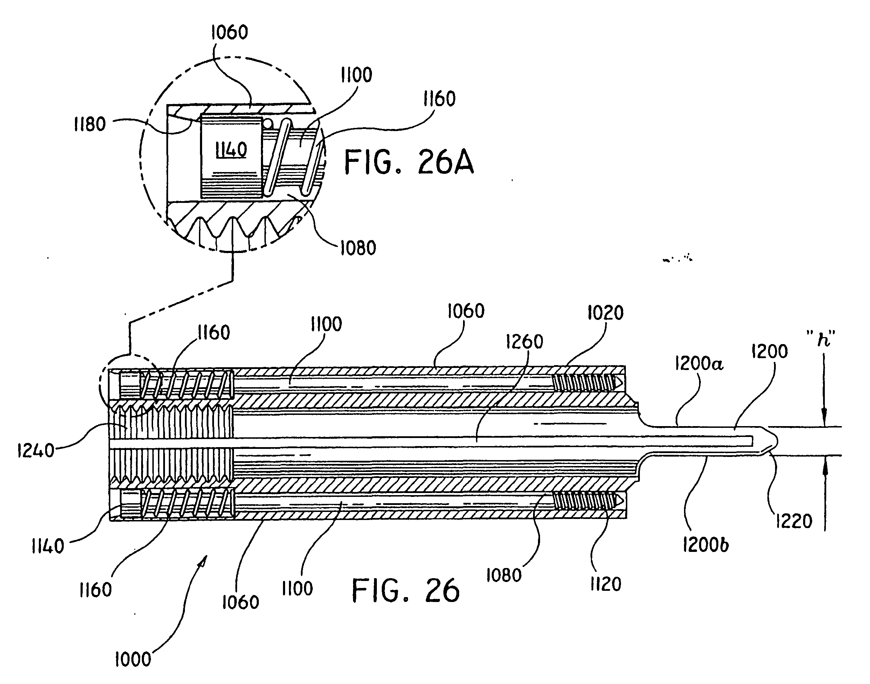

With reference now to FIGS. 25-27, in conjunction with FIG. 24, retractor 1000 includes elongated retractor sleeve 1020 defining a longitudinal axis "a" and having longitudinal opening or passageway 1040 extending therethrough. Retractor sleeve 1020 includes first and second diametrically opposed rails 1060 extending longitudinally along the outer surface of the retractor sleeve 1020. Each rail 1060 has a longitudinal opening 1080. An anchoring member 1100 is disposed within opening 1080 of each rail 1060. Anchoring member 1100 is intended to positively fix retractor 1000 to the bony structures, e..g, adjacent vertebrae. In the preferred embodiment, anchoring member 1100 is in the form of an elongated screw as shown and includes a distal screw thread 1120 which is advantageously configured to penetrate and become mounted within bony tissue. The proximal end of anchoring member 1100 includes structure, e.g., a Phillips head 1140, to be engaged by a driving member, e.g., a Phillips head screw driver or the like, to rotate and advance the anchoring member 1100 in a conventional manner. As depicted in FIG. 26, each anchoring member 1100 is biased proximally by coil spring 1160 whereby distal screw thread 1120 is disposed within opening 1060 of rail 1060 in the unadvanced position of the anchoring member 1100. Anchoring member 1100 is retained within each rail 1060 by lip 1180 extending within opening 1080 of each rail 1060 and engaging the proximal edge of the anchoring member (FIG. 26A), thereby preventing the anchoring member 1100 from exiting the proximal end of retractor sleeve 1020.

Anchoring member 1100 thus constitutes "anchoring means" to positively mount surgical retractor 1000 to the adjacent vertebrae. Other forms of anchoring means are envisioned as well such as, but, not limited to, fasteners, staples, clips etc... which may be driven from the proximal location of retractor sleeve 1020. Additional forms of "anchoring means" may include suture ties, bands, clamps, etc. ...

With reference again to FIGS. 25-27, retractor 1000 includes first and second diametrically opposed retractor arms 1200 extending from the distal end of retractor sleeve 1020. Each retractor arm 1200 has first and second supporting surfaces 1200a, 1200b (FIG. 26) extending in general parallel relation to each other and preferably to the longitudinal axis of retractor sleeve 1020. The height "h" of each arm (i.e., the distance between supporting surfaces 1200a, 1200b) corresponds to the height of the space between adjacent bony structures to be distracted. For example, in spinal implant application, the height "h" of each arm can range from about 0,71 cm (0.28 inches) to about 0.89 cm (0.35 inches). Each arm 1200 further includes tapered end portions 1220 defining a general V-shaped configuration. End portions 1220 facilitate insertion of retractor arms 1200 within the surgical site, e.g., within the intervertebral space.

The proximal end of retractor sleeve 1020 defines an inner threaded bore 1240. Threaded bore 1240 assists in causing translation of the surgical drilling instrument 2000 through retractor sleeve 1020 as will be discussed. Retractor 1000 further includes first and second inner longitudinal recesses 1260 which each extend from the proximal end of retractor sleeve 1020 to an intermediate point of retractor arms 1200. First and second longitudinal recesses 1260 function in maintaining proper alignment of the surgical drilling instrument 2000 inserted within retractor 1000 as will be appreciated from the description provided hereinbelow.

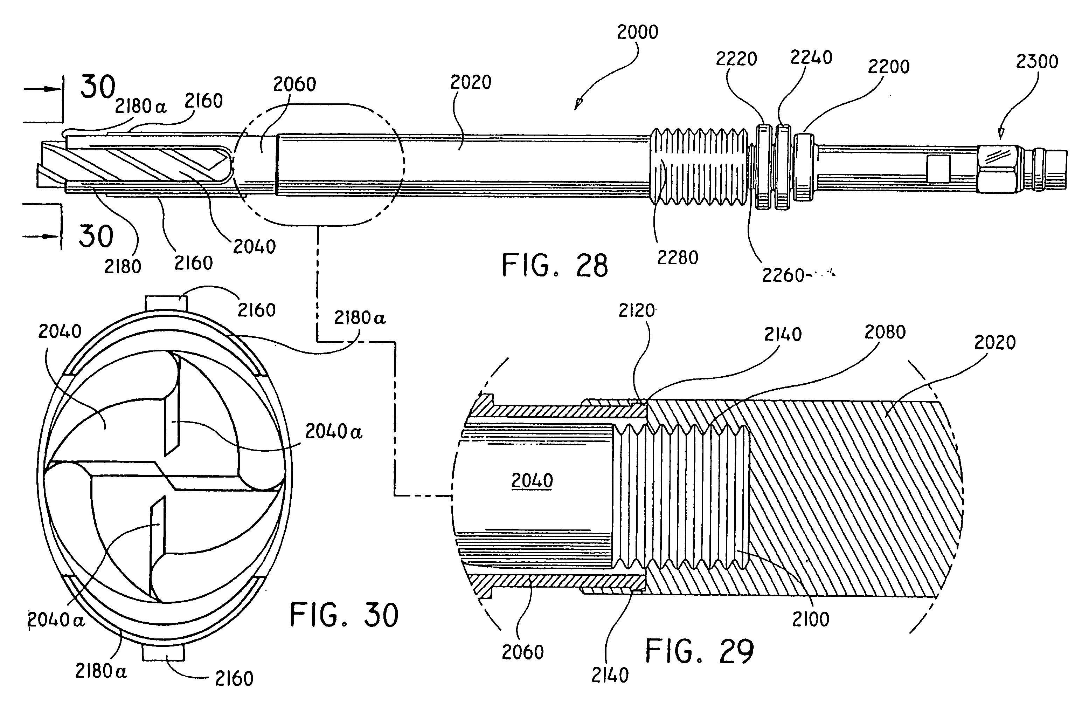

Referring now to FIGS. 28-30, in conjunction with FIG. 24, the surgical drilling instrument 2000 of the system 100 will be discussed. Drilling instrument 2000 is advantageously configured to form an elliptical-shaped bore in the adjacent vertebrae to accommodate the elliptical implant. Clearly, the drill can be configured to form other shaped bores. Drilling instrument 2000 includes drill shaft 2020, drill bit 2040 connected to and extending distally from the drill shaft 2020 and extension sleeve 2060 mounted to the distal end of the drill shaft 2020. In a preferred arrangement, depicted in detail in FIG. 29, drill shaft 2020 includes an internal threaded recess 2080 which threadably engages external threaded portion 2100 of drill bit 2040 to connect the components. With this arrangement, rotational movement of the drill shaft 2020 causes corresponding rotational movement of the drill bit 2040. Drill bit 2040 defines distal cutting edges 2040a which form a generally circular bore in the bone structures.

Extension sleeve 2060 is mounted to drill shaft 2020 to permit relative rotational movement of the two components. In a preferred arrangement, drill shaft 2020 includes a circumferential mounting recess 2120 which receives correspondingly dimensioned circumferential mounting lip 2140 of extension sleeve 2060 in sliding manner to permit rotational movement of the drill shaft 2020 and, thus, rotational movement of the drill bit 2040 within the extension sleeve 2060. Extension sleeve 2060 further defines first and second axial splines 2160 disposed on the outer surface of the extension sleeve 2060 in diametrical arrangement. Axial splines 2160 are received within longitudinal recesses 1260 (FIG. 25) within the interior of the sleeve 2060 to rotationally fix the extension sleeve 2060 within retractor 1000.

Extension sleeve 2060 further defines diametrically opposed cutting arms 2180 at its distal end. Cutting arms 2180 define distal cutting surfaces 2180a which are advantageously dimensioned to cut or shear bony tissue upon advancement of the drill instrument 2000 into the tissue. Cutting surfaces 2180a are preferably arcuate in cross-section as best depicted in FIG. 30. As will be better appreciated hereinbelow, cutting surfaces 2180a in combination with drill bit 2020 form a general elliptical bore in the bony tissue. In particular, drill bit 2020 forms through a drilling action a circular hole while cutting surfaces 2180a cut by a chiseling, shearing action diametrically opposed arcuate sections adjacent the circular bore thereby defining an elliptical configuration of the formed bore in the tissue section.

Referring still to FIGS. 28-30, drill shaft 2020 further includes stationary collar 2200 and first and second movable collars 2220, 2240 adjacent the stationary collar 2200. Moveable collars 2220, 2240 are threadably mounted on threaded portion 2260 and are each moveable on the threaded portion 2260 between a proximalmost position adjacent stationery collar 2200 and a distalmost portion remote from the collar 2200. First collar 2220 serves as a positioning collar, i.e., by adjusting the positioning of first collar 2220 on threaded portion 2260, the depth of penetration of drill shaft 2020 into the bony structures may be adjusted. Second collar 2240 serves as a locking collar to selectively lock the first collar 2220 at a predetermined location on threaded portion 2260.

Drill shaft 2020 further includes an intermediate external threaded portion 2280 disposed at about the midpoint of the drill shaft 2020 to assist in translation of the drill shaft 2020 within the retractor 1000. More particularly, threaded portion 2280 cooperatively threadably engages internal threaded bore 1240 disposed within retractor sleeve 1020. Accordingly, rotation of the drill shaft 2020 causes the drill shaft 2020 to translate longitudinally within the retractor 1000. The proximal end of drill shaft 2020 includes mounting structure 2300, e.g., a hexagonal-shaped head, which cooperates with corresponding structure of a T-shaped handle (to be discussed) to assist in operating the drilling instrument.

FIG. 24A illustrates one type of insertion instrument 4000 utilized to insert the implant 100 within the intervertebral space and a T-shaped handle 5000 utilized to actuate the insertion instrument 4000 and the drilling instrument 2000. Insertion instrument 4000 is disclosed in commonly assigned U.S. patent Application Serial No. 08/616,120, filed March 14, 1996. Insertion instrument 4000 includes implant engaging structure 4020 at its distal end which is correspondingly configured to mount and release implant 100 as will be discussed hereinbelow. A pair of control wheels 4040, 4060 serve to control actuation of insertion instrument 4000 thereby controlling mounting and releasing of the implant within the intervertebral space. T-shaped handle 5000 is mountable to the proximal end of drilling instrument 2000 and to the proximal end of the insertion instrument 4000. Handle 5000 includes hex-head recess 5020. Further details of this instrument 4000 and handle 5000 may be ascertained by reference to the '120 application.

### Use of the System For Insertion of the Fusion Implant

The use of the system 500 for the insertion of the fusion implant 100 into an intervertebral space defined between adjacent lumbar vertebrae will now be described. The subsequent description will be particularly discussed in conjunction with an open antero-lateral approach for spinal fusion implant insertion. However, it is to be appreciated that other approaches, e.g., posterior, direct anterior, etc... could be utilized. Laparoscopic approaches are also envisioned.

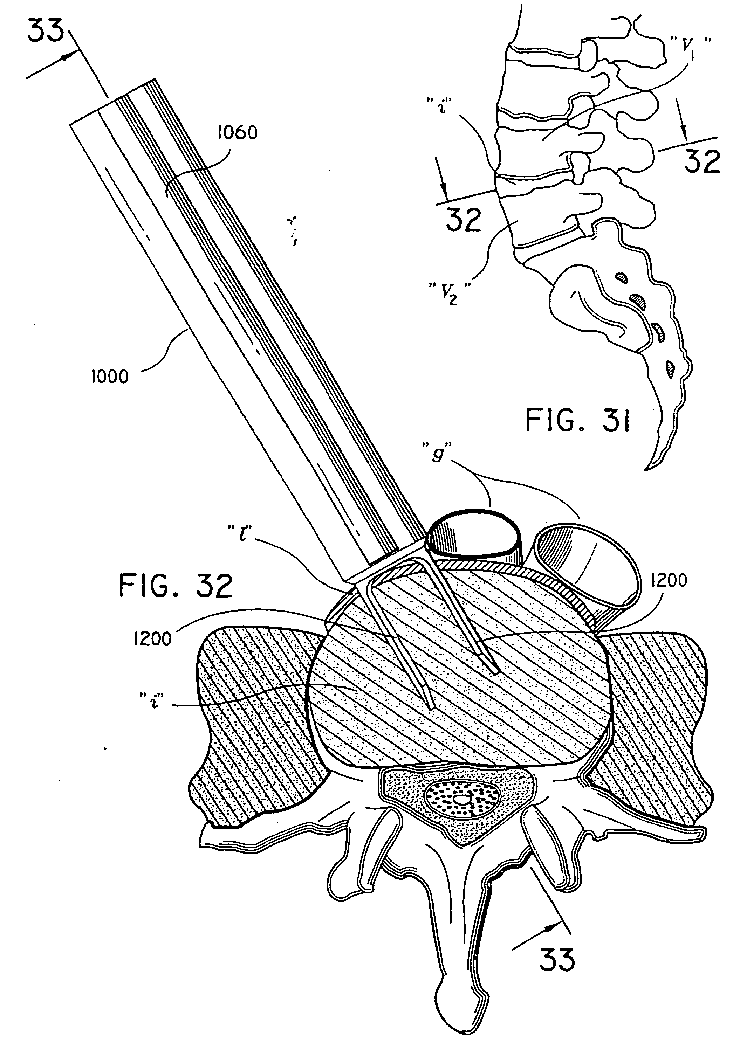

With respect now to FIGS. 31-32, the desired intervertebral space "i" between adjacent vertebrae "v₁, v₂" is accessed utilizing appropriate retractors to expose the anterior vertebral surface. Thereafter, retractor 1000 is inserted within the intervertebral space "i" from an antero-lateral or oblique approach with relation to the vertebral columns "v₁, v₂" as depicted in FIG. 32. Such approach provides advantages with regard to avoiding interference by the great vessels "g", limiting penetration of the anterior longitudinal ligament "l" and minimizing resection of the psoas muscle. The retractor 1000 may be inserted by impacting the proximal end of the retractor to drive the retractor into the intervertebral space.

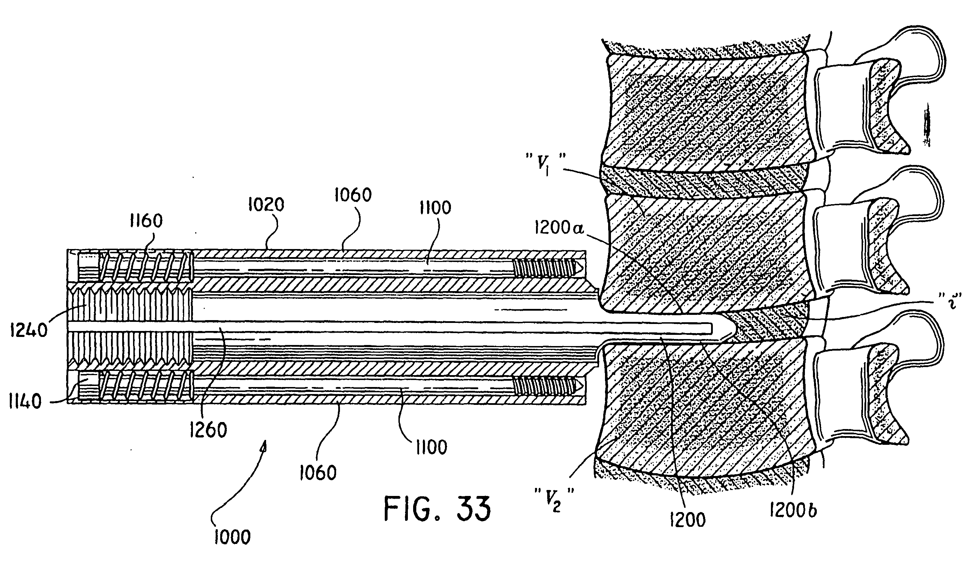

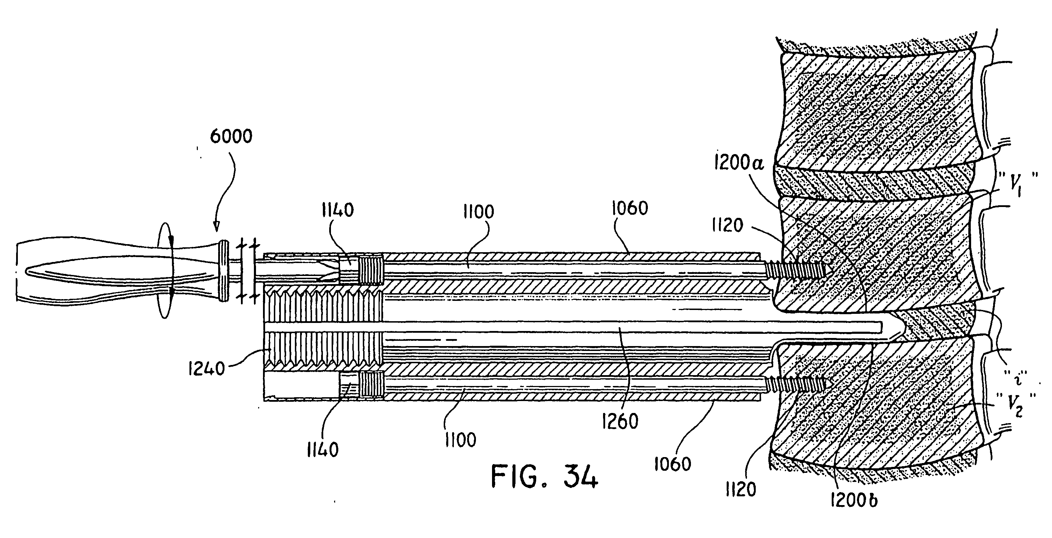

FIG. 33 depicts retractor 1000 positioned within the intervertebral space "i" with the retractor arms 1200 arranged such that the first and second supporting surfaces 1200a, 1200b of each retractor arm 1200 respectively engage the opposed vertebral bodies "v₁, v₂". Upon insertion of retractor arms 1200, the vertebral bodies "v₁, v₂" are distracted whereby the retractor arms 1200 become firmly lodged within the intervertebral space "i". As noted above, upon insertion of the retractor arms 1200, the vertebrae "v₁, v₂" are distracted to a desired operative position. As depicted in FIG. 34, anchoring members 1100 are then advanced within their respective openings 1080 of rails 1060 and mounted within the vertebra "v₁, v₂" with the use of mounting tool 6000, e.g., an elongated driver or the like, whereby the distal screw thread 1120 of each anchoring member engages the vertebral tissue. As a result, retractor 1000 is positively fixed to the vertebral column.

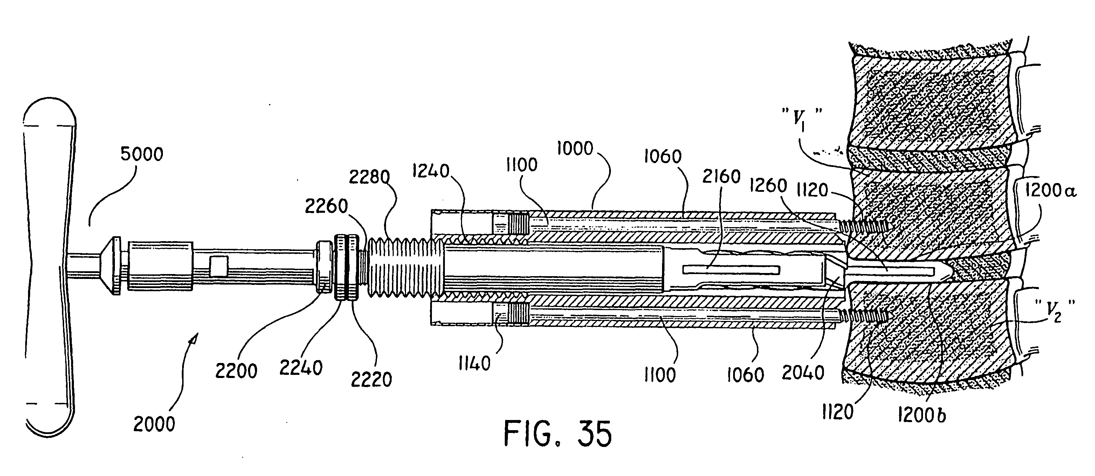

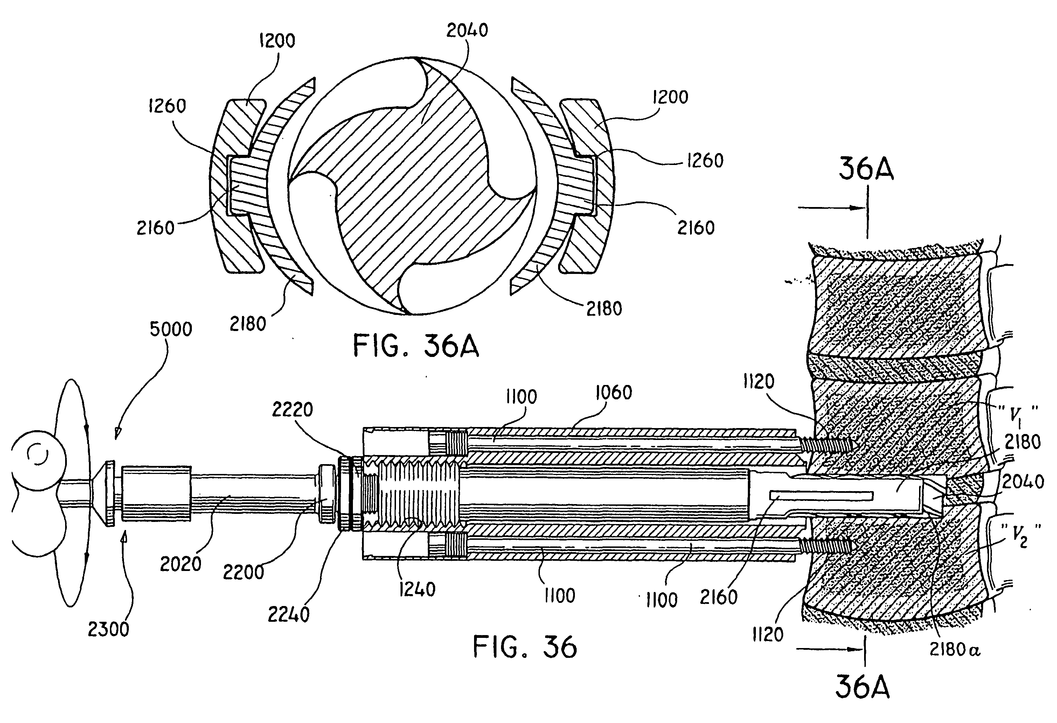

Referring now to FIG. 35, the drilling instrument 2000 is now utilized to prepare the disc space and vertebral end plates for insertion of the fusion implant. The cutting depth of drilling instrument 2000 is adjusted as desired (i.e., to correspond to the length of the fusion implant) by adjusting collars 2220, 2240 of the drilling instrument 2000. In particular, collar 2220 is moved to the desired position on threaded portion 2260 on the drill shaft 2020 and locking collar 2240 is moved adjacent the collar 2220 to lock the collar 2220 at the position. With the T-handle 5000 mounted to drilling instrument 2000, by corresponding reception of hex-head mounting structure 2300 within hex-head bore 5020 of handle 5000, the instrument is introduced into retractor sleeve 1020. Preferably, drill instrument 2000 is inserted within retractor sleeve 1020 whereby axial splines 2160 on the exterior surface of extension sleeve 2060 are received within internal recesses 1260 extending the length of the retractor sleeve 1020 and retractor arms 1200. T-shaped handle 5000 is thereafter rotated which causes drill shaft 2020 and drill bit 2040 to rotate. With reference to FIGS. 36 and 36A, as drill shaft 2020 rotates, it also advances within retractor sleeve 1020 due to the threaded engagement of threaded portion 2280 on the drill shaft 2020 with internal threaded portion 1240 of retractor sleeve 1020 thereby advancing the drill bit 2040 into the adjacent vertebrae "v₁, v₂" to form a circular bore in the end plates of the adjacent vertebrae. In addition, as drill shaft 2020 advances it also drives extension sleeve 2060 distally within the adjacent vertebrae. Due to the interengagement of axial splines 2160 and longitudinal recesses 1260, extension sleeve 2060 advances without rotating whereby cutting surfaces 2180a at the distal end of extension sleeve 2060 cuts through a shearing action into the adjacent vertebrae "v₁, v₂". Thus, the cutting surfaces 2180a of the cutting arms 2180 are retained at the desired angular orientation adjacent retractor arms 1200. The arcuate orientation of the cutting surfaces 2180a of extension sleeve 2060 in combination with drill bit 2040 form a general elliptical opening in the adjacent vertebrae "v₁, v₂". It is to be noted that drilling instrument is advanced within retractor sleeve 1020 until positioning collar 2220 engages the proximal end of the retractor sleeve as shown in FIG. 36 - the length of travel of drilling instrument being predetermined by adjusting collars 2220, 2240 as discussed above.

Subsequent to the drilling process, fusion implant 100 is packed with bone growth inducing substances as is conventional in the art and end cap 104 is threaded into a threaded recess of implant body 102. The fusion implant 100 is then mounted on insertion instrument 4000 by cooperative engagement of the engaging structure 4020 of the insertion instrument with the implant 100 as discussed above.

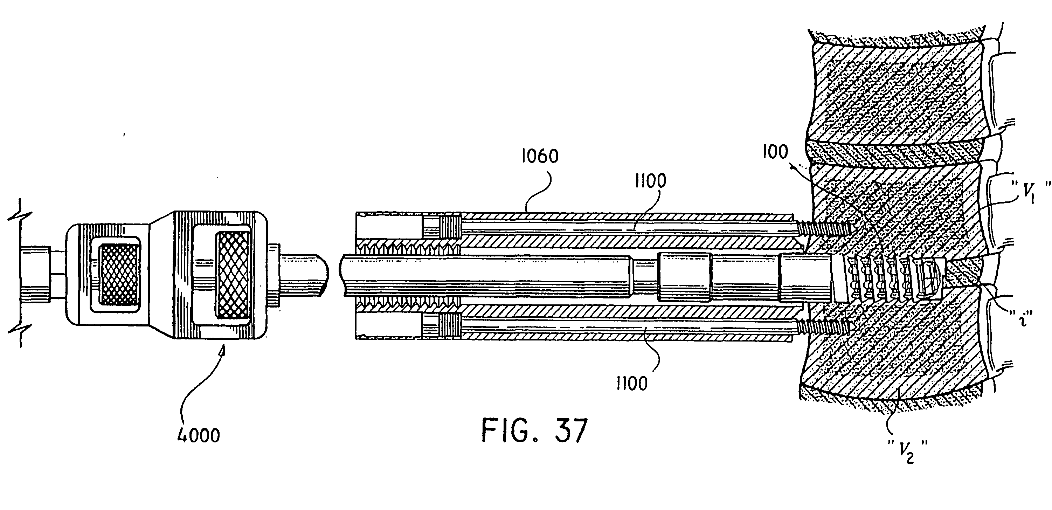

Referring now to FIG. 37, insertion instrument 4000 and mounted implant 100 is introduced within retractor 1000 and advanced to a position adjacent the vertebral bodies "v₁, v₂". Thereafter, insertion instrument 4000 is rotated via T-shaped handle 5000 which is mounted to the instrument 4000 to thereby cause corresponding rotation of fusion implant 3000. As fusion implant 3000 rotates, the thread 3080 of the implant body 3020 bites into the vertebral bodes "v₁, v₂". Continued rotation of insertion tool 4000 causes implant 3000 to be self-tapped within the preformed bore. Implant 3000 is released from its mounting to insertion tool 4000 and the instrument 4000 and retractor 1000 are removed from the disc area.

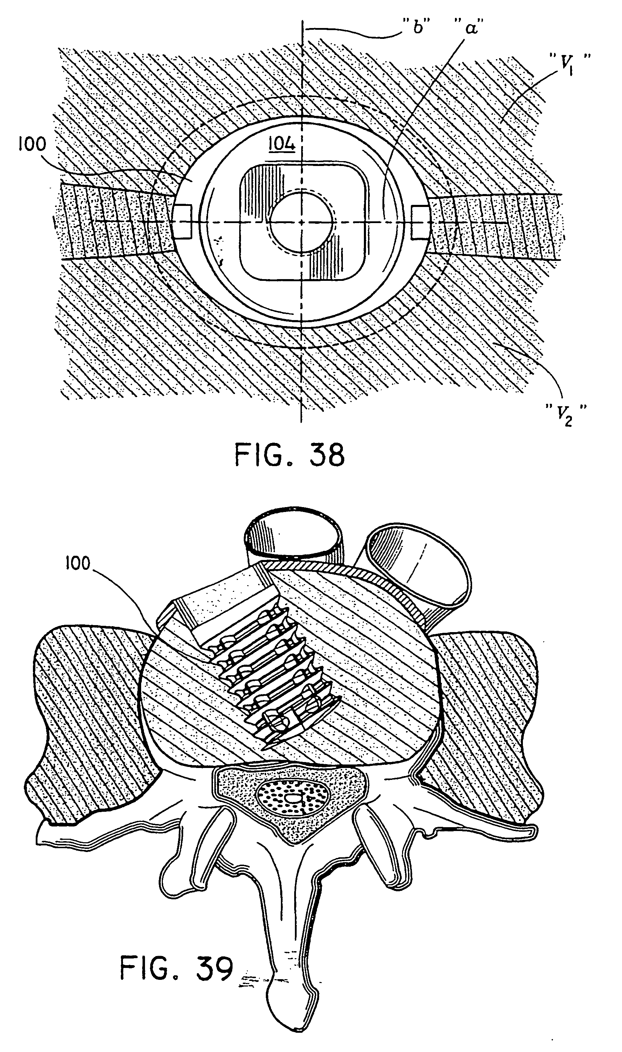

FIGS. 38-39 depict fusion implant 100 inserted within the intervertebral space "i". As shown, fusion implant 100 forms a strut across the intervertebral space "i" to maintain the adjacent vertebrae "v₁, v₂" in appropriate spaced relation during the fusion process. The implant is thus preferably inserted at an angle of between about 15 degrees and about 45 degrees, and more preferably at about 30 degrees to the longitudinal axis of the spine and to the right of the great vessels as view anteriorly. As also shown, in the inserted position of implant 100, the major axis "a" is in general parallel relation to the vertebral end plates thus presenting the great arc or surface area of implant body 102 to contact and support the adjacent vertebrae. Over a period of time, the adjacent vertebral tissue communicates through apertures 112 with the bone growth inducing substances within the interior cavity of implant 100 to form a solid fusion. Thus only one implant is required.

While the above description contains many specifics, these specifics should not be construed as limitations on the scope of the disclosure, but merely as exemplifications of preferred embodiments thereof. For example, the fusion implant 100, 100' could also be used for thoracic and cervical vertebrae. Those skilled in the art will envision many other possible variations that are within the scope of the disclosure as defined by the claims appended hereto.

## Claims

1. A system for drilling a bore in adjacent vertebrae to facilitate the insertion of a fusion implant, comprising a surgical retractor (1000) including a sleeve member (1020) having proximal and distal end portions and defining a longitudinal opening (1040), the distal end portion configured for insertion at least partially into an intervertebral space between adjacent opposed vertebrae to distract the adjacent vertebrae, and a drill instrument (2000) positionable within the longitudinal opening (1040) of the surgical retractor (1000) and including a drill member having a distal cutting head (2040), characterized in that the sleeve member (1020) of the surgical retractor (1000) includes an internal threaded portion (1240) and in that the drill instrument (2000) includes an external threaded portion (2280) engageable with the internal threaded portion (1240) of the retractor (1000) whereby rotation of the drill instrument (2000) causes distal translation of the drill instrument (2000) relative to the surgical retractor (1000).

2. The system according to claim 1 wherein the distal end portion (122) of the sleeve member (1020) of the surgical retractor (1000) includes two spaced apart retractor arms (1200) having first and second supporting surfaces.

3. The system according to claim 1, the drill instrument including:
- an elongate member defining at least one distal cutting surface; and
- a drill member disposed within the elongate member and having a distal cutting head, the drill member being rotatably movable within the elongate member and being longitudinally fixed to the elongate member such that advancement of the drill member within the adjacent vertebrae causes corresponding advancement of the elongate member such that the distal cutting surface and the distal cutting head cooperate to cut a bore in the adjacent vertebrae.

4. The system according to claim 3, wherein the distal end portion of the sleeve member of the surgical retractor includes two spaced apart retractor arms having first and second supporting surfaces.

5. The system according to claim 4, wherein the elongate member of the drill instrument includes first and second diametrically opposed distal cutting surfaces.

6. The system according to claim 3, 4 or 5, including alignment means for aligning and maintaining the elongate member of the drill instrument at a predetermined angular orientation within the sleeve member of the surgical retractor.

7. The system according to claim 6, wherein the alignment means includes at least one groove defined in the sleeve member of the surgical retractor, the at least one groove dimensioned to accommodate a corresponding spine of the elongate member.

8. The system according to any one of the preceding claims, including at least one anchoring member associated with the elongate member and moveable relative to the elongated member to facilitate mounting to vertebrae.

9. The system according to any one of the preceding claims, wherein the longitudinal opening of the sleeve member of the surgical retractor extends substantially the length of the sleeve member.

10. The system according to any one of the claims 3, 4, 5, 6 or 7, wherein the drill member including a distal cutting head, wherein the drill member is operatively connected to the elongate member such that rotation and advancement of the drill member causes corresponding advancement of the elongate member such that the one distal cutting head surface of the elongate member and the distal cutting head of the drill head cooperate to form a substantially elliptical bore in the bony tissue upon advancement therein.

11. The system according to claim 10, wherein the elongate member includes first and second diametrically opposed distal cutting surfaces.

12. The system according to claim 11, wherein the distal cutting surfaces are arcutely shaped.

## Patentansprüche

1. System zum Bohren eines Loches in benachbarte Wirbel, um das Einsetzen eines Verbindungsimplantates zu vereinfachen, welches einen chirurgischen Retraktor (1000), der ein Hülsenelement (1020) aufweist, welches proximale und distale Endabschnitte aufweist und eine longitudinale Öffnung (1040) definiert, wobei der distale Endabschnitt so ausgestaltet ist, um mindestens teilweise in einen Zwischenwirbelraum zwischen benachbarten gegenüberliegenden Wirbeln eingeführt zu werden, um die benachbarten Wirbel zu spreizen, und ein Bohrinstrument (2000) umfasst, welches innerhalb der longitudinalen Öffnung (1040) des chirurgischen Retraktors (1000) positionierbar ist und ein Bohrelement umfasst, welches einen distalen Schneidkopf (2040) aufweist, **dadurch gekennzeichnet, dass** das Hülsenelement (1020) des chirurgischen Retraktors (1000) einen inneren Gewindeabschnitt (1240) umfasst und dass das Bohrinstrument (2000) einen äusseren Gewindeabschnitt (2280) umfasst, der mit dem inneren Gewindeabschnitt (1240) des Retraktors (1000) in Eingriff bringbar ist, wobei durch Rotation des Bohrinstrumentes (2000) ein distaler Versatz des Bohrinstrumentes (2000) relativ zu dem chirurgischen Retraktor (1000) bewirkt wird.

2. System nach Anspruch 1, bei dem der distale Endabschnitt (122) des Hülsenelementes (1020) des chirurgischen Retraktors (1000) zwei räumlich voneinander getrennte Retraktorarme (1200) umfasst, die erste und zweite Unterstützungsflächen aufweisen.

3. System nach Anspruch 1, bei dem das Bohrinstrument umfasst:
- ein längliches Element, welches mindestens eine distale Schneidoberfläche definiert und
- ein Bohrelement, welches innerhalb des länglichen Elementes angeordnet ist und einen distalen Schneidkopf aufweist, wobei das Bohrelement in drehbarer Weise innerhalb des länglichen Elementes bewegbar ist und in longitudinaler Weise mit dem länglichen Element verbunden ist, dass ein Vorschieben des Bohrelementes innerhalb der benachbarten Wirbel ein entsprechendes Vorrücken des länglichen Elementes derart bewirkt, dass die distale Schneidoberfläche und der distale Schneidkopf zusammenwirken, um eine Bohrung in dem benachbarten Wirbel zu bohren.

4. System nach Anspruch 3, bei dem der distale Endabschnitt des Hülsenelementes des chirurgischen Retraktors zwei räumlich voneinander getrennte Retraktorarme umfasst, die erste und zweite Unterstützungsflächen aufweisen.

5. System nach Anspruch 4, bei dem das längliche Element des Bohrinstrumentes erste und zweite diametral gegenüberliegende distale Schneidoberflächen umfasst.

6. System nach Anspruch 3, 4 oder 5, welches Ausrichtungsmittel zum Ausrichten und Halten des länglichen Elementes des Bohrinstrumentes in einer vorbestimmten Winkelausrichtung innerhalb des Hülsenelementes des chirurgischen Retraktors gewährleistet.

7. System nach Anspruch 6, bei dem das Ausrichtungsmittel mindestens eine Nut umfasst, die in dem Hülsenelement des chirurgischen Retraktors definiert ist, wobei die mindestens eine Nut so dimensioniert ist, um einen entsprechenden Wirbel des länglichen Elementes aufzunehmen.

8. System nach einem der vorstehenden Ansprüche, welches mindestens ein Ankerelement umfasst, welches dem länglichen Element zugeordnet ist und in relativer Weise zu dem länglichen Element beweglich ist, um das Befestigen an dem Wirbel zu vereinfachen.

9. System nach einem der vorstehenden Ansprüche, bei dem sich die longitudinale Öffnung des Hülsenelementes des chirurgischen Retraktors im Wesentlichen über die Länge des Hülsenelementes erstreckt.

10. System nach einem der vorstehenden Ansprüche 3, 4, 5, 6 oder 7, bei dem das Bohrelement einen distalen Schneidkopf umfasst, wobei das Bohrelement in operativer Weise mit dem länglichen Element verbunden ist, so dass eine Rotation und ein Vorschieben des Bohrelementes ein entsprechendes Vorschieben des länglichen Elementes derart bewirkt, dass die eine distale Schneidkopfoberfläche des länglichen Elementes und der distale Schneidkopf des Bohrkopfes zusammenwirken, um eine im wesentlichen elliptische Bohrung in dem Knochengewebe bei einem Vorschieben in diesem auszubilden.

11. System nach Anspruch 10, bei dem das längliche Element erste und zweite diametral gegenüberliegende distale Schneidflächen aufweist.

12. System nach Anspruch 11, bei dem die distalen Schneidflächen bogenförmig ausgebildet sind.

## Revendications

1. Système pour percer un trou dans des vertèbres adjacentes pour faciliter l'introduction d'un implant de fusion, comprenant un rétracteur chirurgical (1000), possédant un membre de douille (1020) qui possède des sections terminales, proximales et distales, et définissant une ouverture longitudinale (1040), où la section terminale distale est configurée pour être insérée au moins partiellement dans un espace intervertébral entre des vertèbres adjacentes opposées pour écarter les vertèbres adjacentes, et un instrument de perçage (2000) qui peut être positionné à l'intérieur de l'ouverture longitudinale (1040) du rétracteur chirurgical (1000) et comprend un élément de perçage qui possède une tête de coupe distale (2040), **caractérisé en ce que** le membre de douille (1020) du rétracteur chirurgical (1000) comprend une section taraudée interne (1240) et **en ce que** l'instrument de perçage (2000) comprend une section taraudée externe (2280), qui peut engager la section taraudée interne (1240) du rétracteur (1000), où la rotation de l'instrument de perçage (2000) provoque la translation distale de l'instrument de perçage (2000) relatif au rétracteur chirurgical (1000).

2. Système selon la revendication 1, où la section terminale distale (122) de l'élément de douille (1020) du rétracteur chirurgical (1000) comprend deux bras de rétracteur (1200), l'un dans une distance par rapport à l'autre, comprenant des premières et secondes surfaces de support.

3. Système selon la revendication 1, où l'instrument de perçage comprend:
- un élément oblong, définissant au moins une surface de coupe distale; et
- un élément de perçage disposé à l'intérieur de l'élément oblong et comprenant une tête de coupe distale, où l'élément de perçage peut être déplacé d'une manière rotative à l'intérieur de l'élément oblong et peut être fixé d'une manière longitudinale à l'élément oblong pour que l'avancement de l'élément de perçage à l'intérieur de la vertèbre adjacente cause une avance correspondante de l'élément oblong de telle façon que la surface de coupe distale et la tête de coupe distale coopèrent pour couper un trou dans la vertèbre adjacente.

4. Système selon la revendication 3, où la section terminale distale de l'élément de douille du rétracteur chirurgical comprend deux bras de rétracteur, l'un dans une distance par rapport à l'autre, comprenant des premières et secondes surfaces de support.

5. Système selon la revendication 4, où l'élément oblong de l'instrument de perçage comprend des premières et secondes surfaces de coupe distale diamétrales opposées.

6. Système selon la revendication 3, 4 ou 5, comprenant des moyens d'alignement pour aligner et maintenir l'élément oblong de l'instrument de perçage à une orientation prédéterminée angulaire à l'intérieur de l'élément de douille du rétracteur chirurgical.

7. Système selon la revendication 6, où le moyen d'alignement comprend au moins une rainure définie dans l'élément de douille du rétracteur chirurgical, où au moins cette rainure est dimensionnée pour accommoder une vertèbre correspondante à l'élément oblong.

8. Système selon une quelconque des revendications précédentes, comprenant au moins un élément d'ancrage associé au membre oblong et qui peut être déplacé relatif à l'élément oblong pour faciliter le montage à la vertèbre.

9. Système selon une quelconque des revendications précédentes, où l'ouverture longitudinale de l'élément de douille du rétracteur chirurgical s'étend essentiellement sur la longueur de l'élément de douille.

10. Système selon une quelconque des revendications 3, 4, 5, 6 où 7, où l'élément de perçage comprend une tête de coupe distale, où l'élément de perçage est attaché d'une manière opérationnelle à l'élément oblong pour que la rotation et l'avance de l'élément de perçage causent une avance correspondante à l'élément oblong de tel façon que la surface de tête coupante distale de l'élément oblong et la tête de coupe distale de la tête de perçage coopèrent pour former un trou essentiellement elliptique dans le tissu osseux pendant leur avance dans celui-ci.

11. Système selon la revendication 10, où l'élément oblong comprend des premières et secondes surfaces de coupe distale diamétralement opposées.

12. Système selon la revendication 11, où les surfaces de coupe distales sont arquées.
